# EUROPÄISCHE PATENTSCHRIFT

(11) **EP 1 192 470 B1**
(45) Veröffentlichungstag und Bekanntmachung des Hinweises auf die Patenterteilung: **08.09.2004**
(21) Anmeldenummer: 00943453.1
(22) Anmeldetag: 21.06.2000
(51) Int. Cl.: G01N 33/68, C07K 14/47

(54) **VERFAHREN ZUR FERTILITÄTSBESTIMMUNG VON SÄUGETIEREN, INSBESONDERE VON MENSCHEN**
METHOD FOR DETERMINING THE FERTILITY OF MAMMALS, ESPECIALLY HUMANS
PROCEDE POUR DETERMINER LA FERTILITE DE MAMMIFERES, NOTAMMENT CHEZ L'HUMAIN

(30) Priorität: 25.06.1999 AT 111999
(43) Veröffentlichungstag der Anmeldung: 03.04.2002
(73) Patentinhaber: Vitateq Biotechnology GmbH, 6020 Innsbruck (AT)
(72) Erfinder: ILLMENSEE, Karl, Oskar, A-6020 Innsbruck (AT); DIEPLINGER, Hans, A-6020 Innsbruck (AT)
(74) Vertreter: Alge, Daniel, Mag. Dr. rer.nat.
(86) Internationale Anmeldenummer: PCT/AT2000/000171
(87) Internationale Veröffentlichungsnummer: WO 2001/001148

(56) Entgegenhaltungen:
- WO-A-95/27059
- BIOLOGICAL ABSTRACTS, Philadelphia PA USA; abstract no. PREV199900389820, abstract XP002153018 & L. JERKOVIC L ET AL: "Afamin and vitamin E in follicular fluid of patients undergoing IVF" HUMAN REPRODUCTION , Bd. 14, Nr. Abstr. Book 1, 1. Juni 1999 (1999-06-01), Seiten 203-204, Oxford UK

## Beschreibung

Die Erfindung betrifft ein Verfahren zur Fertilitätsbestimmung von Säugetieren, insbesondere von Menschen.

Bedingt durch die Zunahme an Wissen und Fertigkeiten in der in vitro-Fertilisationstechnik ist der Bedarf an verlässlichen und auch kostengünstigen Fertilisationsbestimmungsmethoden stark gestiegen. Dabei geht es aber nicht nur um die prinzipielle Diagnose von Fertilisationsstörungen, sondern in zunehmendem Maße auch um die Detektion von Fertilisationsgraden und die Bestimmung der Befruchtung und/oder Einnistung der befruchteten Oozyten.

Afamin ist ein 87 kDa-Protein, welches zur Albumingruppe zählt und viele strukturelle und biochemische Gemeinsamkeiten mit den Proteinen dieser Gruppe, wie z.B. mit humanem Serumalbumin, humanem α-Fetoprotein oder humanem Vitamin D-Bindungsprotein, aufweist. Afamin wurde bereits kloniert und sequenziert und steht daher auch in rekombinanter Form zur Verfügung (WO 95/27059).

Darüber hinaus ergaben biochemische und physiologische Untersuchungen, dass dieses Protein Vitamin E-bindende Eigenschaften aufweist. Es konnte nachgewiesen werden, dass Afamin nicht nur im Blut, sondern auch in anderen Körper- oder Organflüssigkeiten, wie beispielsweise Cerebrospinal-Follikel und Samenflüssigkeit, auftritt.

Die Aufgabe der vorliegenden Erfindung liegt im Zurverfügungstellen einer völlig neuartigen Fertilitätsbestimmungsmethode.

Diese Aufgabe wird erfindungsgemäß gelöst durch ein Verfahren zur Fertilitätsbestimmung von Säugetieren, insbesondere von Menschen, welches sich dadurch auszeichnet, dass
- der Afamingehalt einer einem Säugetier entnommenen Körperoder Organflüssigkeit bestimmt wird und
- der ermittelte Afamingehalt mit einem Referenzwert zur Bestimmung der Fertilität verglichen wird.

Die vorliegende Erfindung beruht auf der überraschenden Erkenntnis, dass die Afaminkonzentration in den verschiedenen Körperoder Organflüssigkeiten, wo Afamin vorhanden ist, direkt mit Fertilitätseigenschaften korreliert. Diese Korrelation beschränkt sich nicht nur auf das Vorhandensein von Fertilitätsstörungen, sondern ist auch zur Diagnose von Fertilitätsschwankungen oder Schwangerschaften möglich. Erfindungsgemäß hat sich sogar gezeigt, dass neben der Detektion von Anwesenheit oder Abwesenheit von Oozyten über die erfindungsgemäße Methodologie zur Bestimmung des Afamingehaltes sogar eine Aussage hinsichtlich des Reifungsgrades von Oozyten möglich ist.

Die vorliegende Erfindung kann in der Humanmedizin, insbesondere bei der Überwachung von in vitro-Fertilisationen oder bei Fertilisationsdiagnosen und -gutachten, eingesetzt werden. Sie hat aber auch enorme Einsatzmöglichkeiten im Rahmen der modernen Tierzucht, da sie sich in einfacher Weise standardisieren lässt und keine komplizierten Laboreinrichtungen zur Durchführung des Tests erforderlich sind.

Afamin ist in vielen verschiedenen Körper- oder Organflüssigkeiten vorhanden und es hat sich erfindungsgemäß gezeigt, dass der Afamingehalt in all diesen Flüssigkeiten mit den Fertilitätseigenschaften korreliert. Gemäß bevorzugten Ausführungsformen der vorliegenden Erfindung wird der Afamingehalt aber vorwiegend in Körper- oder Organflüssigkeiten bestimmt, die sich durch einen hohen physiologischen Afamingehalt auszeichnen, wie z.B. Serum, Follikel- oder Samenflüssigkeit. Es ist aber selbstverständlich auch möglich, das erfindungsgemäße Verfahren mit anderen Körperoder Organflüssigkeiten, wie etwa Cerebrospinalflüssigkeit, durchzuführen, da auch die Konzentration von Afamin in diesen anderen Flüssigkeiten in Bereichen liegt, die hinsichtlich der möglichen Afamin-Nachweisgrenze in der Regel keine Probleme mit sich bringt.

Die Art und Weise, wie das erfindungsgemäße Verfahren in der Praxis durchgeführt wird, ist vollkommen beliebig, vor allem, was die Art der Entnahme der Körper- oder Organflüssigkeit oder die Bestimmung des Afamingehaltes anbelangt. Beispielsweise kann der Afamingehalt immunologisch, elektrophoretisch oder chromatographisch bestimmt werden. Immunologische Bestimmungsverfahren für Afamin werden erfindungsgemäß oft bevorzugt, da nicht nur eine Reihe verschiedener monoklonaler Antikörper gegen verschiedenste Epitope von Afamin zur Verfügung stehen, sondern weil sich gerade immunologische Tests, etwa in Form von Standard-ELISA-Tests leicht derart konzipieren lassen, dass sie auch ohne aufwendiges Laborinstrumentarium durchgeführt und ausgewertet werden können (etwa in Kombination mit kolorimetrischen Nachweisverfahren). Dadurch ist es möglich, das erfindungsgemäße Bestimmungsverfahren auch in einer für Laien durchführbaren Form zur Verfügung zu stellen.

Als Referenzwert wird üblicherweise ein Afamin-Normalwert für die jeweilige Körper- oder Organflüssigkeit herangezogen. Dieser kann beispielsweise in Form von Vergleichswerten, Vergleichskurven oder Vergleichstabellen beim erfindungsgemäßen Verfahren herangezogen oder - was generell bevorzugt wird - durch gleichzeitige Bestimmung einer Referenzprobe (mit definiertem Afamingehalt) zusammen mit der Probe der entnommenen Körper- oder Organflüssigkeit erhalten werden. In letzterem Fall wird der wahrscheinlich nie ganz auszuschaltende systematische Fehler, den unterschiedliche Bestimmungsmethoden bzw. unterschiedliche Bestimmungsbedingungen mit sich bringen können, von vornherein hintangehalten. Dies kann vor allem bei Bestimmungen, wo es nur um graduelle Unterschiede im Afamingehalt geht (etwa bei Bestimmung des Reifungsgrades von Oozyten), wichtig sein.

Bevorzugterweise wird die erfindungsgemäß vermessene Probe nicht nur mit einem Referenzwert, sondern mit zwei oder mehreren Referenzwerten verglichen. So kann man beispielsweise neben einem "Normalwert" auch einen anderen Referenzwert bzw. eine Referenzprobe vorsehen, wie etwa eine "pathologische" Referenz oder eine "Schwangerschafts"-Referenz, usw.

Erfindungsgemäß bevorzugt ist aber jedenfalls das Vorsehen eines Referenzwertes für den Afamingehalt in der entsprechenden Körper- oder Organflüssigkeit, die dem Afaminwert eines Normalpatienten (oder im Falle der Tierzucht der Probe des normalen Tieres) entspricht.

Dieser Referenzwert wird bei der Durchführung des erfindungsgemäßen Verfahrens bevorzugterweise dadurch erhalten, dass parallel zur Fertilitätsbestimmung der Probe der Afamingehalt einer Referenzprobe ermittelt wird.

Erfindungsgemäß bevorzugt wird die Bestimmung des Afamingehaltes mittels immunologischer Methoden, insbesondere unter Verwendung eines monoklonalen Antikörpers, da hierdurch die Standardisierung sehr effizient zu erreichen ist und auch für verschiedenste Chargen eines Bestimmungskits die Kompatibilität der ermittelten Daten zueinander gegeben ist.

Es hat sich erfindungsgemäß gezeigt, dass insbesondere in der Samen- und Follikelflüssigkeit neben dem Afamingehalt auch der Vitamin E-Gehalt direkt mit der Fertilitätseigenschaft korreliert und daher ebenfalls für das erfindungsgemäße Verfahren herangezogen werden kann. Bevorzugterweise wird daher das erfindungsgemäße Verfahren weiters mit einer Bestimmung des Vitamin E-Gehaltes in der jeweiligen Körper- oder Organflüssigkeit verbunden, welcher Vitamin E-Gehalt dann gegebenenfalls auch mit einem Referenzwert verglichen wird.

Verfahren zur Bestimmung von Vitamin E in Körper- oder Organflüssigkeiten sind mannigfaltig beschrieben. Wie bei der Bestimmung des Afamingehaltes im Rahmen der vorliegenden Erfindung, ist auch die spezifische Art und Weise, wie der Vitamin E-Gehalt bestimmt wird, für die vorliegende Erfindung nicht kritisch, jedoch sind HPLC oder andere biochemische Methoden die bevorzugten Methoden für die Bestimmung von Vitamin E-Konzentrationen.

Ein weiterer Aspekt der vorliegenden Erfindung bezieht sich auf die Verwendung eines Kits, umfassend
· eine Probe einer Körper- oder Organflüssigkeit eines Säugetieres oder ein Gefäß zur Aufnahme der Körper- oder Organflüssigkeit,
· ein Reagens zur Detektion des Afamingehaltes in der Probe, und
· Afamin-Referenzmittel,
zur Bestimmung der Fertilität von Säugetieren.

Wie oben erwähnt, ist die Wahl des Reagens zur Detektion des Afamingehaltes selbstverständlich abhängig von der jeweiligen Detektionsmethodik. Beispielsweise umfasst das Reagens zur Detektion des Afamingehaltes vorzugsweise einen Antikörper gegen Afamin, insbesondere einen monoklonalen Afamin-Antikörper. Bevorzugterweise weist dieser Afamin-Antikörper auch weitere Nachweismittel, wie etwa fluoreszierende, radioaktive oder chromogene Gruppen auf, oder kann durch andere Detektionsmittel gebunden werden (z.B. durch Sekundär-Antikörper).

Das Afamin-Referenzmittel umfasst bevorzugterweise eine standardisierte Menge an Afamin, etwa eine Referenzprobe der jeweiligen Körper- oder Organflüssigkeit. Andererseits kann das Afamin-Referenzmittel aber auch aus einem einfachen Vergleichswert oder einer Vergleichstabelle bzw. einer Vergleichskurve bestehen, die bevorzugterweise für die jeweilige Körper- oder Organflüssigkeit und die jeweilige Nachweismethodik standardisiert ist.

Besonders bevorzugt ist ein erfindungsgemäßer Kit, welcher eine ganze Reihe von standardisierten Afaminproben aufweist, die z.B. eine Eichgerade definieren oder für bestimmte Fertilisationsmerkmale repräsentativ sind.

Wenn mit dem erfindungsgemäßen Kit auch der Vitamin E-Gehalt der jeweiligen Körper- oder Organflüssigkeit bestimmt werden soll, sind selbstverständlich auch die für diese Messung erforderlichen Reagentien und Referenzmittel (letztere sind beim Vorsehen von Referenzproben natürlich identisch) erforderlich.

Gemäß einem weiteren Aspekt betrifft die vorliegende Erfindung eine Fertilitätsbestimmung durch Bestimmen des Vitamin E-Gehaltes von Samen- und Follikel-Flüssigkeit und die Korrelation dieses Gehaltes mit einem entsprechenden Referenzwert, sowie einen entsprechenden Kit zur Durchführung dieses Verfahrens.

Das erfindungsgemäße Verfahren bzw. der erfindungsgemäß Kit werden bevorzugterweise zur Überwachung von Patienten im Rahmen von Fertilisierungsverfahren, insbesondere bei in vitro-Fertilisierung und intrazytoplasmischer Spermien-Injektion, verwendet. Hierbei ist es erforderlich, bei Testpersonen sehr genau die Anwesenheit oder Abwesenheit von Oozyten sowie deren Qualität für eine erfolgreiche Befruchtung bzw. die Funktionalität von Spermien mit einem einfachen Test routinemäßig zu überwachen.

Eine weitere bevorzugte Verwendung des erfindungsgemäßen Tests bzw. der erfindungsgemäßen Kits liegt in der Bestimmung des Reifegrades von Oozyten oder Spermien.

Weiters kann das erfindungsgemäße Verfahren bzw. der erfindungsgemäße Kit zur Untersuchung von Fertilitäts- und Reproduktionsstörungen verwendet werden und ist insbesondere für breite Reihentests und zur systematischen Untersuchung von großen Personengruppen sehr gut geeignet.

Eine weitere besondere Anwendungsmöglichkeit des erfindungsgemäßen Verfahrens bzw. des erfindungsgemäßen Kits liegt in der Überwachung von Schwangerschaften, beginnend mit der Festellung des Vorliegens einer Schwangerschaft (als Schwangerschaftstest) sowie nachfolgend mit der Überwachung der Schwangerschaft, insbesondere dem Risiko von Beeinträchtigungen von oxidativen oder radikalen Substanzen, bedingt durch einen reduzierten Gehalt von Afamin bzw. Vitamin E.

Die vorliegende Erfindung wird an Hand der nachfolgenden Beispiele und der Zeichungsfiguren näher erläutert. Es zeigen:
Fig. 1 : die Korrelation von Afamin in Follikel-Flüssigkeit von Personen mit oder ohne Eizelle;
Fig. 2 : die Korrelation von Afamin-Konzentration und Follikelgröße;
Fig. 3 : die Korrelation von Afamin und α-Tocopherol-Konzentration in Follikel-Flüssigkeit;
Fig. 4 : die Korrelation von Afamin und Reifungsgrad von Oozyten in Follikel-Flüssigkeit und Serum, aufgeschlüsselt nach großen, mittleren und kleinen Oozyten;
Fig. 5 : SDS-PAGE mit Immunoblotting von 9 Proben Follikel-Flüssigkeit;
Fig. 6 : SDS-PAGE mit Immunoblotting von 3 Proben liquor cerebrospinalis.

### Beispiele :

Patienten die einer Ovulationsinduktion für in vitro-Fertilisierung (IVF) oder intrazytoplasmatischer Spermien-Injektion (ICSI) unterzogen wurden, wurden Follikel verschiedener Größe mittels ultrasonographischer Mittel individuell punktiert. Die individuellen Proben der Follikel-Flüssigkeit wurden auf Anwesenheit oder Abwesenheit von Oozyten getestet, zentrifugiert und zur weiteren Untersuchung gelagert. Blutproben dieser Patienten wurden am Tag der Follikelpunktion entnommen und für die Serumaufarbeitung gesammelt. Die Konzentration von Afamin in der Follikel-Flüssigkeit und im Serum wurde quantitativ mittels Sandwich-ELISA (unter Verwendung von monoklonalen Antikörpern) gemessen. Die Bestimmung von Vitamin E in diesen Proben wurde nach Proteinfällung und reversed-phased HPLC vorgenommen. Qualitative Analyse von Afamin in Follikel-Flüssigkeit und Serum wurde mittels SDS-PAGE und Immunoblotting vorgenommen.

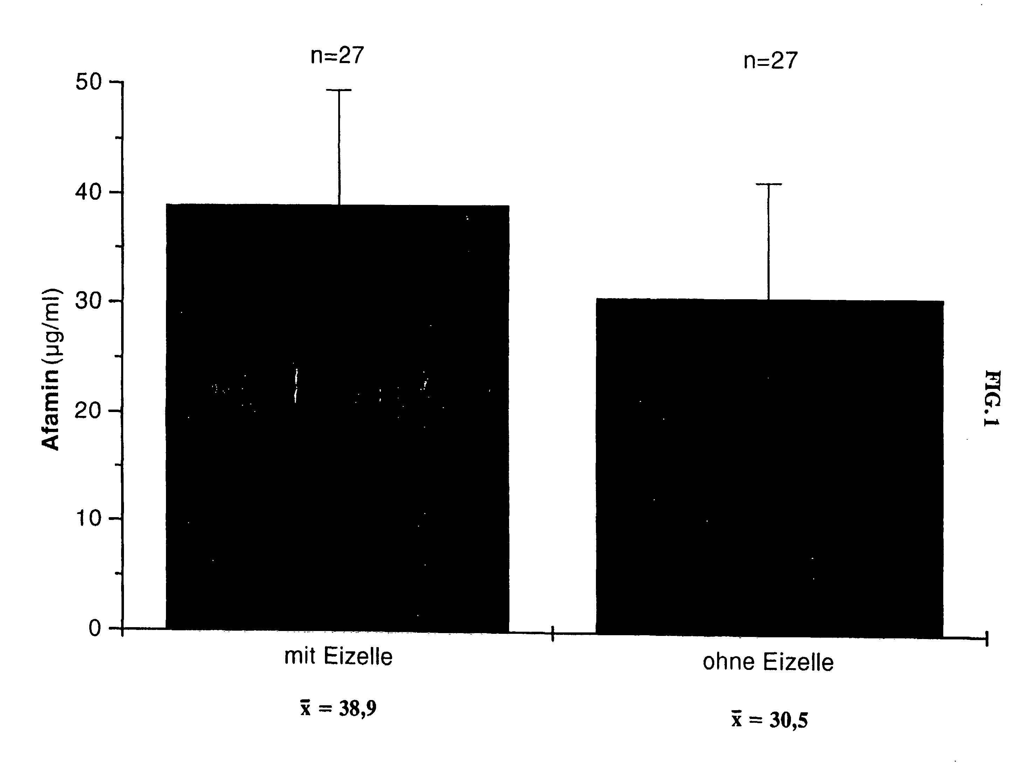

Zunächst wurden jeweils 27 Proben von Patienten mit bzw. ohne Eizelle entnommen und der Afamingehalt ermittelt. Es zeigte sich, dass die Proben von Patienten mit Eizelle eine mittlere Afaminkonzentration von 38,9 µg/ml aufwiesen, die Proben von Patienten ohne Eizelle jedoch nur 30,5 (Fig. 1). Es zeigte sich also, dass die Afaminkonzentration bei Patienten mit Eizelle um nur 30 % höher lag, als der "Normalwert" (= ohne Eizelle).

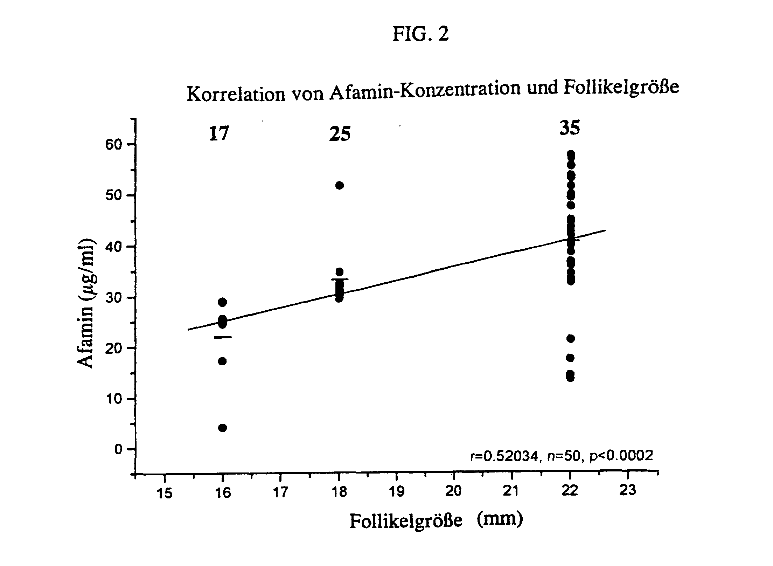

Im Anschluß daran wurde untersucht, ob und wie die Größe des Follikels mit der Afaminkonzentration korreliert. Dabei wurden die Follikel in 3 verschiedene Größentypen klassifiziert und in Fig. 2 gegen die ermittelte Afaminkonzentration aufgetragen. Es zeigte sich, dass auch hierbei eine eindeutige Korrelation hervortritt, so dass die Afaminkonzentration ein direktes Maß für den Reifungsgrad einer Eizelle darstellt.

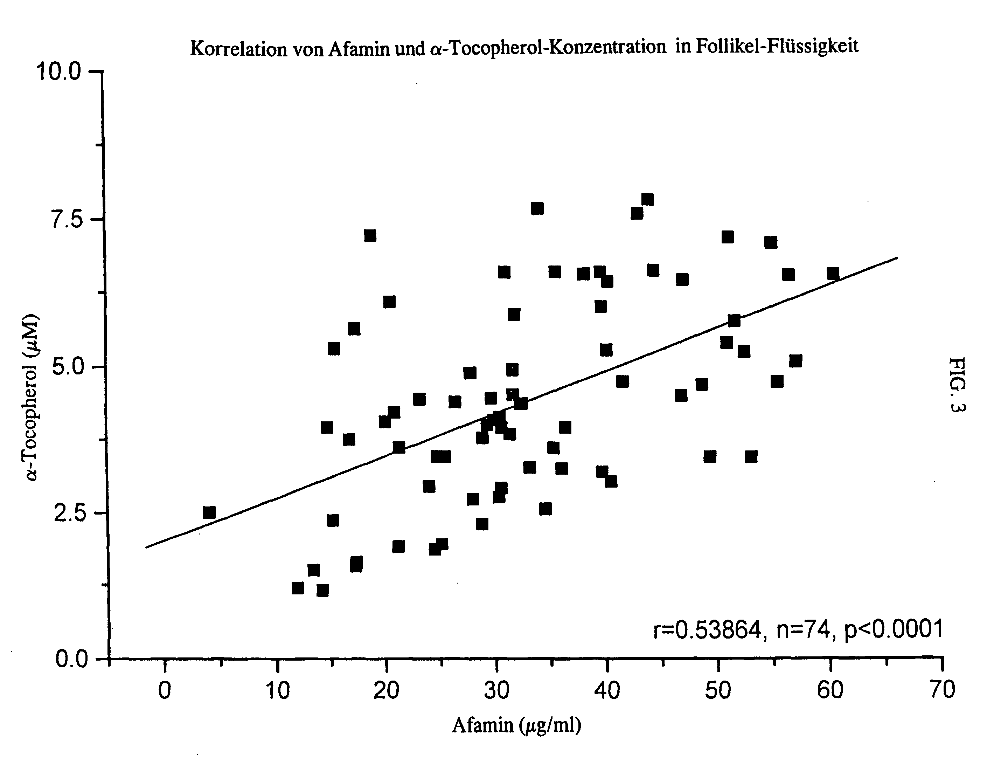

Aus Fig. 3 ergibt sich, dass auch eine eindeutige Korrelation zwischen der Vitamin E-Konzentration und der Afaminkonzentration in der Follikelflüssigkeit besteht, d.h., je höher die Afaminkonzentration ist, desto höher liegt die Vitamin E-Konzentration.

In einer weiteren Versuchsreihe wurde getestet, ob die für Follikelflüssigkeit erwiesene Korrelation zwischen An-/Abwesenheit von Eizellen bzw. den verschiedenen Reifungsgraden der Oozyten auch z.B. in Serum gegeben ist.

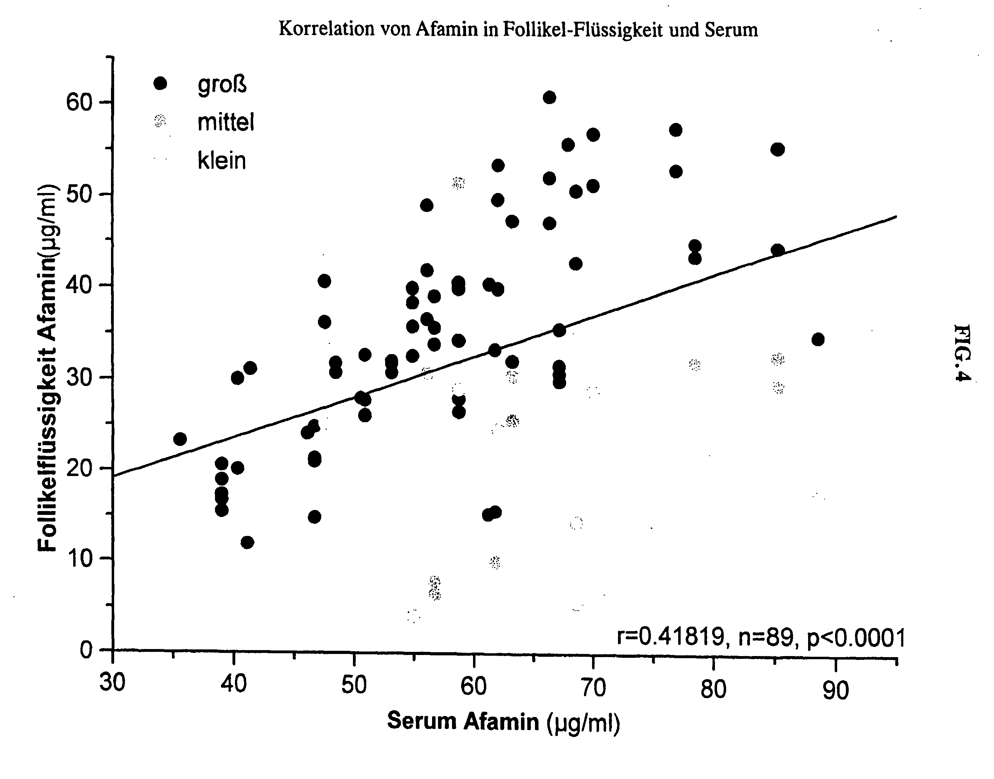

Wie aus Fig. 4 ersichtlich ist, können auch in Serum die verschiedenen Reifungsgrade von Eizellen über den Afamingehalt bestimmt werden.

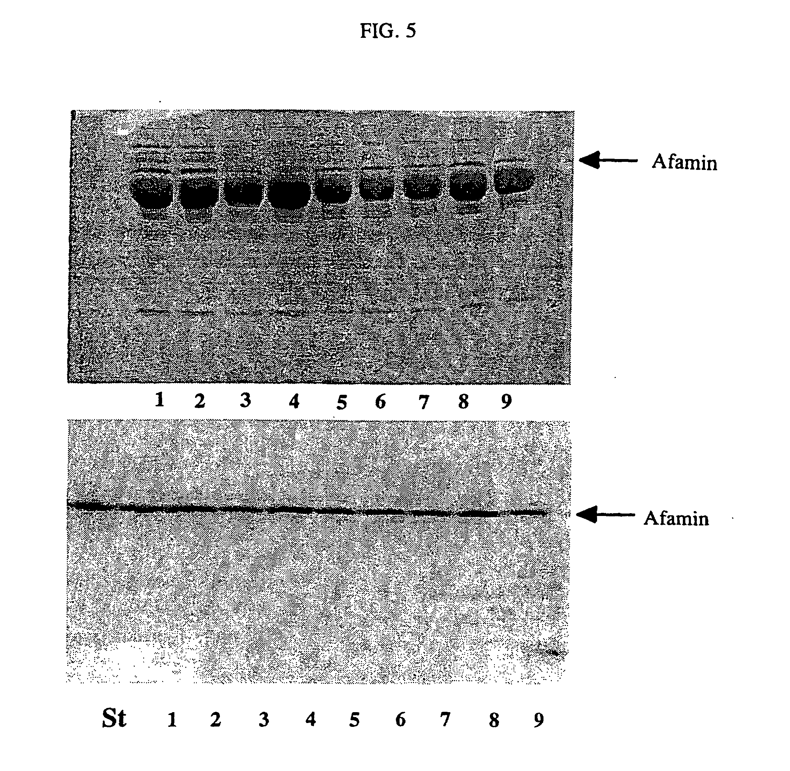

In Fig. 5 ist eine Reihe von 9 verschiedenen Proben durch SDS-PAGE und Immunoblotting mit einem monoklonalen Afamin-Antikörper dargestellt. Hierbei kann die Bestimmung von Afamin über den Standard (ganz links in Fig. 5) vorgenommen werden.

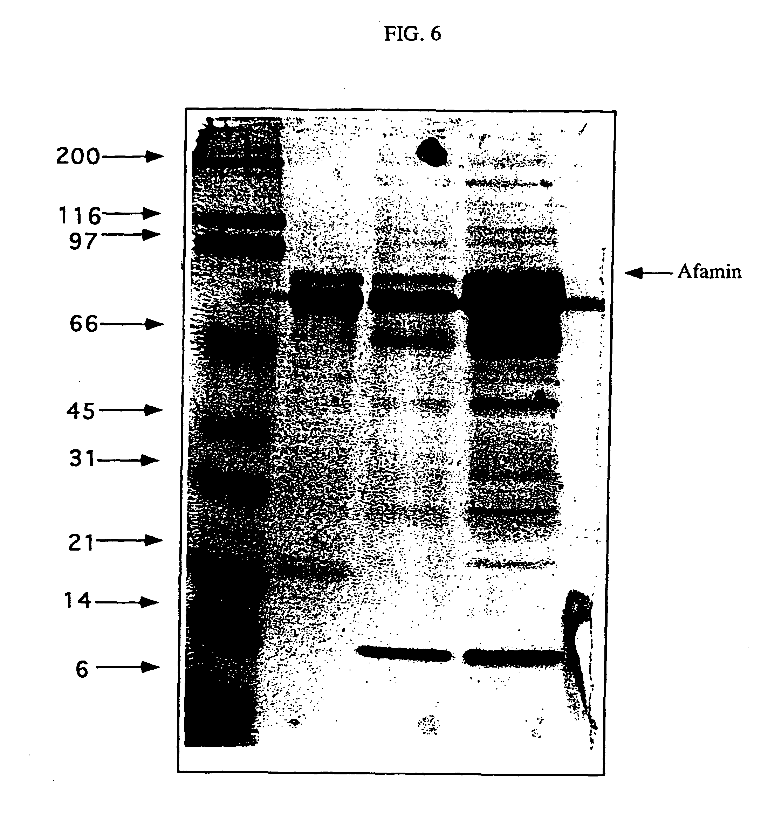

Schließlich wurde auch getestet, ob das erfindungsgemäße System auch in Cerebrospinal-Flüssigkeit Anwendung finden kann (Fig. 6). Wie sich aus dieser Fig. 6 ergibt, lassen sich auch dort aufgrund der Varianz in den Afamin-Gehalten eindeutige Aussagen über die Fertilitätseigenschaften treffen.

## Patentansprüche

1. Verfahren zur Fertilitätsbestimmung von Säugetieren, insbesondere von Menschen, **dadurch gekennzeichnet, dass**
· der Afamingehalt einer einem Säugetier entnommenen Körper- oder Organflüssigkeit bestimmt wird und
· der ermittelte Afamingehalt mit einem Referenzwert verglichen wird.

2. Verfahren nach Anspruch 1, **dadurch gekennzeichnet, dass** die Körper- oder Organflüssigkeit Serum, Follikel- oder Samenflüssigkeit ist.

3. Verfahren nach Anspruch 1 oder 2, **dadurch gekennzeichnet, dass** der Referenzwert der Afamingehalt in der entsprechenden Körper- oder Organflüssigkeit eines Normalpatienten ist.

4. Verfahren nach einem der Ansprüche 1 bis 3, **dadurch gekennzeichnet, dass** der Referenzwert parallel zur Fertlitätsbestimmung ermittelt wird.

5. Verfahren nach einem der Ansprüche 1 bis 4, **dadurch gekennzeichnet, dass** der Afamingehalt mittels immunologischer Methoden, insbesondere unter Verwendung eines monoklonalen Antikörpers, ermittelt wird.

6. verfahren nach einem der Ansprüche 1 bis 5, **dadurch gekennzeichnet, dass** zusätzlich der Vitamin E-Gehalt in der Körperoder Organflüssikeit bestimmt und gegebenenfalls mit einem Referenzwert verglichern wird.

7. Verwendung eines Kits, umfassend
· eine Probe einer Körper- oder Organflüssigkeit eines Säugetieres oder ein Gefäß zur Aufnahme der Körper- oder Organflüssigkeit
· ein Reagens zur Detektion des Afamingehaltes in der Probe,
· ein Afamin-Referenzmittel
zur Bestimmung der Fertilität von Säugetieren.

8. Verwendung nach Anspruch 7, **dadurch gekennzeichnet, dass** das Reagens zur Detektion des Afamingehaltes einen Antikörper, insbesondere einen monoklonalen Antikörper, enthält.

9. Verwendung nach Anspruch 7 oder 8, **dadurch gekennzeichnet, dass** das Afamin-Referenzmittel eine standardisierte Menge an Afamin ist.

10. Verwendung nach einem der Ansprüche 7 bis 9, **dadurch gekennzeichnet, dass** der Afamin-Standard eine Reihe von standardisierten Afamin-Proben ist.

11. Verwendung eines Verfahrens nach einem der Ansprüche 1 bis 6 oder eines Kits, wie in einem der Ansprüche 7 bis 10 definiert, zur Überwachung von Patienten im Rahmen von Fertilisierungsverfahren, insbesondere bei in vitro-Fertilisierung und intrazytoplasmischer Spermien-Injektion.

12. Verwendung eines Verfahrens nach einem der Ansprüche 1 bis 6 oder eines Kits, wie in einem der Ansprüche 7 bis 10 definiert, zur Bestimmung des Reifegrades von Oozyten oder Spermien.

13. Verwendung eines Verfahrens nach einem der Ansprüche 1 bis 6 oder eines Kits, wie in einem der Ansprüche 7 bis 10 definiert zur Untersuchung von Fertilitäts- und Reproduktionsstörungen.

14. Verwendung eines Verfahrens nach einem der Ansprüche 1 bis 6 oder eines Kits, wie in einem der Ansprüche 7 bis 10 definiert, zur Überwachung von Schwangerschaften.

## Claims

1. A method for determining the fertility of mammals, in particular of humans, **characterised in that**
· the afamin content of a body or organ fluid taken from a mammal is determined, and
· the determined afamin content is compared with a reference value.

2. A method according to claim 1, **characterized in that** the body or organ fluid is serum, follicular or seminal fluid.

3. A method according to claim 1 or 2, **characterized in that** the reference value is the afamin content in the corresponding body or organ fluid of a normal patient.

4. A method according to any one of claims 1 to 3, **characterized in that** the reference value is determined in parallel to the fertility determination.

5. A method according to any one of claims 1 to 4, **characterized in that** the afamin content is determined by immunological methods, in particular by using a monoclonal antibody.

6. A method according to any one of claims 1 to 5, **characterized in that** the vitamin E content is additionally determined in the body or organ fluid and optionally is compared to a reference value.

7. The use of a kit comprising
· a sample of a body or organ fluid from a mammal, or a vessel for receiving the body or organ fluid,
· a reagent for detecting the afamin content in the sample, and
· afamin reference means
for determining the fertility of mammals.

8. The use according to claim 7, **characterized in that** the reagent for detecting the afamin content comprises an antibody, in particular a monoclonal antibody.

9. The use according to claim 7 or 8, **characterized in that** the afamin reference means is a standardized amount of afamin.

10. The use according to any one of claims 7 to 9, **characterized in that** the afamin standard is a series of standardized afamin samples.

11. The use of a method according to any one of claims 1 to 6 or of a kit as defined in any one of claims 7 to 10 for monitoring patients within the scope of fertilization methods, in particular in in vitro fertilization and intracytoplasmatic sperm injection.

12. The use of a method according to any one of claims 1 to 6 or of a kit as defined in any one of claims 7 to 10 for determining the degree of maturity of oocytes or sperms.

13. The use of a method according to any one of claims 1 to 6 or of a kit as defined in any one of claims 7 to 10 for investigating fertility and reproductive disturbances.

14. The use of a method according to any one of claims 1 to 6 or of a kit as defined in any one of claims 7 to 10 for monitoring pregnancies.

## Revendications

1. Procédé pour déterminer la fertilité de mammifères, en particulier des êtres humains, **caractérisé en ce que**
• on détermine la teneur en afamine d'un liquide corporel ou d'un liquide d'organe prélevé sur un mammifère et
• on compare la teneur déterminée en afamine à une valeur de référence.

2. Procédé selon la revendication 1, **caractérisé en ce que** le liquide corporel ou le liquide d'organe est du sérum, du liquide folliculaire ou du liquide séminal.

3. Procédé selon la revendication 1 ou 2, **caractérisé en ce que** la valeur de référence est la teneur en afamine dans le liquide corporel ou le liquide d'organe correspondant d'un patient normal.

4. Procédé selon l'une des revendications 1 à 3, **caractérisé en ce qu'**on détermine la valeur de référence parallèlement à la détermination de la fertilité.

5. Procédé selon l'une des revendications 1 à 4, **caractérisé en ce qu'**on détermine la teneur en afamine au moyen de procédés immunologiques, en particulier en utilisant un anticorps monoclonal.

6. Procédé selon l'une des revendications 1 à 5, **caractérisé en ce qu'**on détermine en outre la teneur en vitamine E dans le liquide corporel ou le liquide d'organe et, le cas échéant, on la compare à une valeur de référence.

7. Utilisation d'un kit comprenant
• un échantillon d'un liquide corporel ou d'un liquide d'organe d'un mammifère ou un récipient pour recevoir le liquide corporel ou le liquide d'organe
• un réactif pour la détection de la teneur en afamine dans l'échantillon
• un moyen de référence d'afamine
pour la détermination de la fertilité de mammifères.

8. Utilisation selon la revendication 7, **caractérisée en ce que** le réactif pour la détection de la teneur en afamine contient un anticorps, en particulier un anticorps monoclonal.

9. Utilisation selon la revendication 7 ou 8, **caractérisée en ce que** le moyen de référence d'afamine est une quantité normalisée d'afamine.

10. Utilisation selon l'une des revendications 7 à 9, **caractérisée en ce que** la norme d'afamine est une série d'échantillons normalisés d'afamine.

11. Utilisation d'un procédé selon l'une des revendications 1 à 6 ou d'un kit tel que défini dans l'une des revendications 7 à 10 pour la surveillance de patients dans le cadre de procédés de fertilisation, en particulier pour une fertilisation in vitro et une injection intracytoplasmique de spermatozoïdes.

12. Utilisation d'un procédé selon l'une des revendications 1 à 6 ou d'un kit tel que défini dans l'une des revendications 7 à 10 pour la détermination du degré de maturation d'ovocytes ou de spermatozoïdes.

13. Utilisation d'un procédé selon l'une des revendications 1 à 6 ou d'un kit tel que défini dans l'une des revendications 7 à 10 pour l'examen des troubles de la fertilité et de la reproduction.

14. Utilisation d'un procédé selon l'une des revendications 1 à 6 ou d'un kit tel que défini dans l'une des revendications 7 à 10 pour la surveillance de grossesses.
